# EUROPEAN PATENT SPECIFICATION

(11) **EP 0 928 792 B1**
(45) Date of publication and mention of the grant of the patent: **19.04.2006**
(21) Application number: 99300073.6
(22) Date of filing: 06.01.1999
(51) Int. Cl.: C07D 333/24, A61K 31/415, A61K 31/44, A61K 31/38, C07C 229/50, C07D 213/55, C07D 277/30, C07D 233/64

(54) **Bicyclic(3.1.0)hexanes and related compounds**
Bicyclische(3.1.0)hexane und verwandte Verbindungen
Hexanes (3.1.0) bicycliques et composés apparentés

(30) Priority: 08.01.1998 US 70759 P
(43) Date of publication of application: 14.07.1999
(73) Proprietor: Pfizer Products Inc., Groton, Connecticut 06340 (US)
(72) Inventor: Chenard, Bertrand Leo, Waterford, Connecticut 06385 (US)
(74) Representative: Hayles, James Richard

(56) References cited:
- EP-A- 0 696 577
- WO-A-97/17952

## Description

### Background of the Invention

The present invention relates to compounds of the formula I, as described below, their pharmaceutically acceptable salts, pharmaceutical compositions containing them and their use in treating neurological and psychiatric disorders.

The role of excitatory amino acids, such as glutamic acid and aspartic acid, as the predominant mediators of excitatory synaptic transmission in the oentral nervous system has been well established. Watkins & Evans, *Ann. Rev. Pharmacol. Toxicol.,* 21, 165 (1981); Monaghan, Bridges, and Cotman, *Ann. Rev. Pharmacol. Toxicol.,* 29. 365 (1989); Watkins, Krogsgaard-Larsen, and Honore, *Trans. Pharm. Sci*., 11, 25 (1990). These amino acids function in synaptic transmission primarily through excitatory amino acid receptors. These amino acids also participate in a variety of other physiological processes such as motor control, respiration, cardiovascular regulation, sensory perception, and cognition.

Excitatory amino acid receptors are classified into two general types. Receptors that are directly coupled to the opening of cation channels in the cell membrane of the neurons are termed "ionotropic." This type of receptor has been subdivided into at least three subtypes, which are defined by the depolarizing actions of the selective agonists N-methyl-D-aspartate (NMDA), alpha-amino-3-hydroxy-5-methylisoxazole-4-propionic acid (AMPA), and kainic acid (KA). The second general type is the G-protein or second messenger-linked "metabotropic" excitatory amino acid receptor. This second type, when activated by the agonists quisqualate, ibotenate, or trans-1-aminocyclopentane-1,3-dicarboxylic acid, leads to enhanced phosphoinosoitide hydrolysis in the postsynaptic cell. Both types of receptors appear not only to mediate normal synaptic transmission along excitatory pathways, but also participate in the modification of synaptic connection during development and changes in the efficiency of synaptic transmission throughout life. Schoepp, Bockaert, and Sladeczek. *Trends in Pharmacol. Sci.,* 11, 508 (1990); McDonald and Johnson, *Brain Research Reviews,* 15, 41 (1990).

The excessive or inappropriate stimulation of excitatory amino acid receptors leads to neuronal cell damage or loss by way of a mechanism known as excitotoxicity. This process has been suggested to mediate neuronal degeneration in a variety of conditions. The medical consequences of such neuronal degeneration makes the abatement of these degenerative neurological processes an important therapeutic goal.

Excitatory amino acid excitotoxicity has been implicated in the pathophysiology of a number of neurological disorders. This excitotoxicity has been implicated in the pathophysiology of acute and chronic neurodegenerative conditions including stroke, cerebral ischemia, spinal cord trauma, head trauma, Alzheimer's Disease, Huntington's Chorea, amyotrophic lateral sclerosis, epilepsy, AIDS-induced dementia, perinatal hypoxia, hypoxia (such as conditions caused by strangulation, surgery, smoke inhalation, asphyxiation, drowning, choking, electrocution or drug or alcohol overdose), cardiac arrest, hypoglycemic neuronal damage, ocular damage and retinopathy, idiopathic and drug-induced Parkinson's Disease and cerebral deficits subsequent to cardiac bypass surgery and grafting. Other neurological conditions that are caused by glutamate dysfunction require neuromodulation. These other neurological conditions include muscular spasms, migraine headaches, urinary incontinence, psychosis, addiction withdrawal (such as alcoholism and drug addiction including opiate, cocaine and nicotine addiction), opiate tolerance, anxiety, emesis, brain edema, chronic and acute pain, convulsions, retinal neuropathy, tinnitus and tardive dyskinesia. The use of a neuroprotective agent, such as an AMPA receptor antagonist, is believed to be useful in treating these disorders and/or reducing the amount of neurological damage associated with these disorders. The excitatory amino acid receptor (EAA) antagonists are also believed to be useful as analgesic agents.

The metabotropic glutamate receptors are a highly heterogeneous family of glutamate receptors that are linked to multiple second-messenger pathways. Generally, these receptors function to modulate the presynaptic release of glutamate, and the postsynaptic sensitivity of the neuronal cell to glutamate excitation. The metabotropic glutamate receptors (mGluR) have been pharmacologically divided into two subtypes. One group of receptors ("Class I receptors") is positively coupled to phospholipase C, which causes hydrolysis of cellular phosphoinositides (PI). This first group are termed PI-linked metabotropic glutamate receptors. The second group of receptors ("Class II receptors") is negatively coupled to adenyl cyclase, which prevents the forskolin-stimulated accumulation of cyclic adenosine monophosphate (cAMP). Schoepp and Conn, Trends Pharmacol. Sci., 14, 13 (1993). Receptors within this second group are termed cAMP-linked metabotropic glutamate receptors. Agonists of the cAMP-linked metabotropic glutamate receptors should be useful for the treatment of acute and chronic neurological conditions and psychiatric conditions.

Compounds have recently been discovered that effect metabotropic glutamate receptors, but have no effect on ionotropic glutamate receptors. (1S,3R)-1-Aminocyclopentane-1,3-dicarboxylic acid (1S,3R-ACPD) is an agonist of PI-linked and cAMP-linked metabotropic glutamate receptors. Schoepp, Johnson, True, and Monn., Eur. J. Pharmacol., 207, 351 (1991); Schoepp, Johnson, and Monn, J. Neurochem., 58, 1184 (1992). (2S,3S,4S)-2-(carboxycyclopropyl) glycine (L-CCG-1) was recently described as a selective cAMP-linked metabotropic glutamate receptor agonist: however, at higher concentrations, this compound has activity at PI-linked metabotropic receptors. Nakagawa, et al., Eur J. Pharmacol., 184, 205 (1990): Hayashi, et al., Br. J. Pharmacol., 197, 539 (1992) Schoepp et al., J. Neurochem., 63., 769-772 (1994).

European Patent Application EP 696577Al, which was published on February 14, 1996, refers to certain synthetic amino acids that are described as being selective for the negatively coupled cAMP linked metabotropic glutamate receptors (i.e., Class II metabotropic glutamate receptors).

The compounds of formula I and their pharmaceutically acceptable salts are metabotropic glutamate receptor ligands that are selective for Class II metabotropic glutamate receptors.

### Summary of the Invention

This invention relates to compounds of the formula 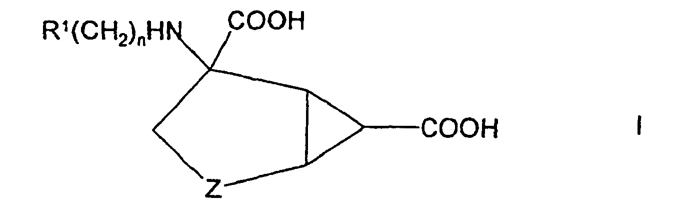 wherein n is 0-6;
Z is (C₁-C₄) alkylene, oxygen, sulfur, NH or N(C₁-C₆)alkyl;
R¹ is hydrogen, (C₁-C₆)alkyl, aryl or heteroaryl, wherein said aryl is selected from phenyl and naphthyl and said heteroaryl is selected from 5 and 6 membered aromatic heterocyclic rings that contain from one to four heteroatoms selected, independently, from nitrogen, oxygen and sulfur, and wherein said aryl and heteraryl moieties can optionally be substituted with one or more substituents, preferably with one or two substituents, that are selected, independently, from halo (e.g., fluoro, chloro, bromo or iodo), -SO(C₁-C₆)alkyl, -SO₂R⁴, -SO₂NR⁵R⁶, (C₁-C₆)alkyl optionally substituted with from one to seven fluorine atoms, (C₁-C₆)alkoxy optionally substituted with from one to seven fluorine atoms, amino, nitro, cyano, carboxy, -CO₂(C₁-C₆)alkyl, (C₁-C₆)alkylamino, di-[(C₁-C₆)alkyl]amino phenoxy, anilino and phenylthio;
R⁴ is -O(C₁-C₆)alkyl, (C₁-C₆)alkyl or phenyl; and
R⁵ and R⁶ are independently selected from hydrogen, (C₁-C₆)alkyl and phenyl;
with the proviso that R¹ can not be hydrogen when n is zero, and with the proviso that none of the foregoing heteroaryl moieties may contain more than one ring oxygen atom or more than one ring sulfur atom;
and the pharmaceutically acceptable salts of such compounds.

Examples of the heteroaryl moieties of said heteroaryl-(C₀-C₆)alkyl are the following: oxazolyl, isoxazoyl, thiazolyl, isothiazolyl, furanyl, pyrazolyl, pyrrolyl, tetrazolyl, triazolyl, thienyl, imidazolyl, pyridinyl, and pyrimidinyl.

This invention also relates to compounds of the formula 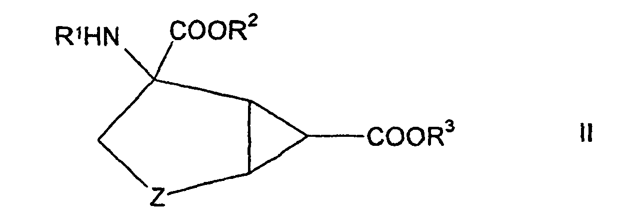 wherein R¹ and Z are defined as for formula I above and R² and R³ are selected, independently, from hydrogen and (C₁-C₆)alkyl, with the proviso that only one of R² and R³ can be hydrogen.

Compounds of the formula 11 are intermediates in the synthesis of compounds of the formula I.

Preferred compounds of the formula I include those wherein R¹ is a pyrid-3-yl or pyrid-4-yl group.

Other examples of preferred compounds of the formula I are those wherein R¹ is linked to the bicyclic ring depicted in structural formula I via an alkyl group.

Other examples of preferred compounds of the formula I are those wherein R¹ is a mono- or disubstituted phenyl group wherein one of the substituents is in the para position.

Other examples of preferred compounds of the formula I are those wherein Z is CH₂.

Other examples of preferred compounds of the formula I are those wherein R¹ is a thien-2-ylmethyl group.

The compounds of formula I and their pharmaceutically acceptable salts are metabotropic glutamate receptor ligands and are useful in the treatment of a variety of neurological and psychiatric disorders. Examples of neurological disorders that can be treated with the compounds of formula I and their pharamaceutically acceptable salts are cerebral deficits subsequent to cardiac bypass surgery and grafting, cerebral ischemia (e.g., stroke and cardiac arrest), spinal cord trauma, head trauma, Alzheimer's Disease, Huntington's disease, amyotrophic lateral sclerosis, AIDS-induced dementia, muscular spasms, migrane headache, urinary incontinence, convulsions, perinatal hypoxia, hypoglycemic neuronal damage, chemical dependencies and addictions (e.g., a dependency on, or addiction to opiates, benzodiazepines, cocaine, nicotine or ethanol), drug or alcohol withdrawal symptoms, ocular damage and retinopathy, cognitive disorders, idiopathic and drug-induced Parkinson's Disease, emesis, brain edema, acute or chronic pain, sleep disorders, Tourette's syndrome, attention deficit disorder and tardive dyskinesia. Examples of psychiatric disorders that can be treated "with the compounds of formula I and their pharamaceutically acceptable salts are schizophrenia, anxiety and related disorders (e.g., panic attack and stress-related disorders), depression, bipolar disorders, psychosis, and obsessive compulsive disorders.

Examples of specific preferred compounds of the formula I are those wherein Z is CH₂ and R¹ is one of the following groups.

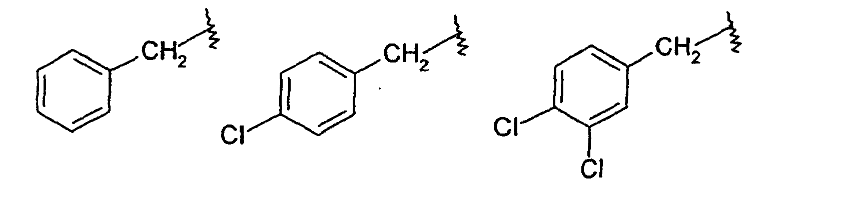

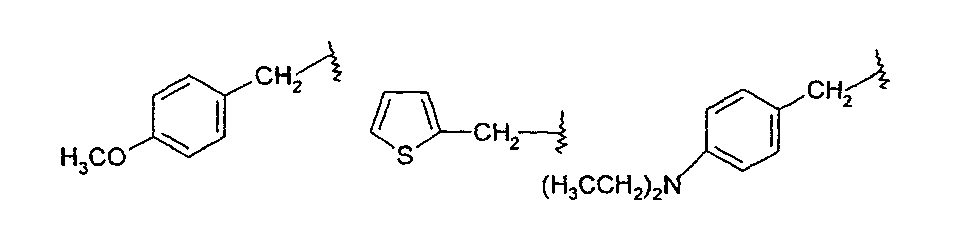

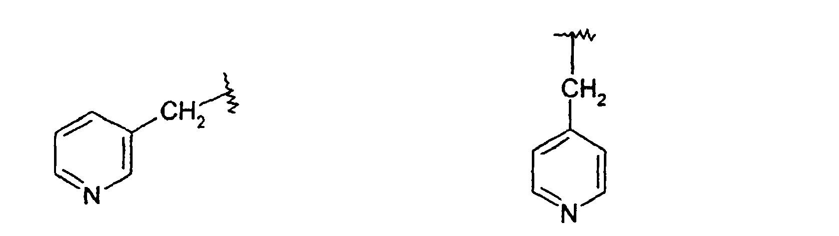

Another example of a specific preferred compound of the formula I is the compound of formula I wherein Z is CH₂ and R¹ is 3-methylbutyl.

Other examples of specific compounds of the formula I are the following:
2-Benzylamino-bicyclo[3.1.0]hexane-2,6-dicarboxylic acid;
2-(4-Methoxy-benzylamino)-bicyclo[3.1.0]hexane-2,6-dicarboxylic acid;
2-(4-Diethylamino-benzylamino)-bicyclo[3.1.0]hexane-2,6-dicarboxylic acid;
2-(4-Chloro-benzylamino)-bicyclo[3.1.0]hexane-2,6-dicarboxylic acid;
2-(3,4-Dichloro-benzylamino)-bicyclo[3.1.0]hexane-2,6-dicarboxylic acid;
2-[(Pyridin-3-ylmethyl)-amino]-bicyclo[3.1.0]hexane-2,6-dicarboxylic acid;
2-[(Pyridin-4-ylmethyl)-amino]-bicyclo[3.1.0]hexane-2,6-dicarboxylic acid;
2-[(Thiophen-2-ylmethyl)-amino]-bicyclo[3.1.0]hexane-2,6-dicarboxylic acid;
2-(4-Pyrrolidin-1-yl-benzylamino)-bicyclo[3.1.0]hexane-2.6-dicarboxylic acid;
2-(4-Piperidin-1-yl-benzylamino)-bicyclo[3.1.0]hexane-2,6-dicarboxylic acid and
2-[(6-Pyrrolidin-1-yl-pyridin-3-ylmethyl)-amino]-bicyclo[3.1.0]hexane-2,6-dicarboxylic acid.

The present invention also relates to the pharmaceutically acceptable acid addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3-naphthoate)]salts.

The present invention also relates to the pharmaceutically acceptable base addition salts of compounds of the forumula I. These salts are all prepared by conventional techniques. The chemical bases that are used as reagents to prepare the pharmaceutically acceptable base salts of this invention are those which form non-toxic base salts with the acidic compounds of formula I. Such non-toxic base salts include those derived from such pharmacologically acceptable cations as sodium, potassium, calcium and magnesium, etc.

This invention also relates to a pharmaceutical composition for treating a disorder or condition, the treatment or prevention of which can be effected or facilitated by modulating (i.e., increasing or decreasing) glutamate neurotransmission in a mammal, comprising an amount of a compound of the formula I, or a pharmaceutically effective salt thereof, that is effective in treating such disorder or condition and a pharmaceutically acceptable carrier.

This invention also relates to compounds of formula I for use in treating a disorder or condition, the treatment of which can be effected or facilitated by modulating glutamate neurotransmission in a mammal, comprising administering to a mammal in need of such treatment an amount of a compound of the formula I, or a phartnaceutically effective salt thereof, that is effective in treating such disorder or condition.

This invention also relates to a pharmaceutical composition for treating a condition selected from stroke, cerebral ischemia, spinal cord trauma, head trauma, Alzheimer's Disease, Huntington's Chorea, amyotrophic lateral sclerosis, epilepsy, AIDS-induced dementia, muscular spasms, migraine headaches, urinary incontinence, psychosis, convulsions, perinatal hypoxia, hypoxia (such as conditions caused by strangulation, surgery, smoke inhalation, asphyxiation, drowning, choking, electrocution or drug or alcohol overdose), cardiac arrest, hypoglycemic neuronal damage, chemical dependencies and addictions (e.g., addictions to or dependencies on alcohol, opiates, benzodiazepines, nicotine, heroin or cocaine), ocular damage, retinopathy, retinal neuropathy, tinnitus, idiopathic and drug induced Parkinson's Disease, anxiety, panic disorder, schizophienia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting in a mammal, comprising a glutamate neurotransmission modulating effective amount of a compound of the formula I, or a pharmaceutically salt thereof, and a pharmaceutically acceptable carrier.

This invention also relates to compounds of formula I for use in treating a condition selected from stroke, cerebral ischemia, spinal cord trauma, head trauma, Alzheimer's Disease, Huntington's Chorea, amyotrophic lateral sclerosis, epilepsy, AIDS-induced dementia, muscular spasms, migraine headaches, urinary incontinence, psychosis, convulsions, perinatal hypoxia, hypoxia (such as conditions caused by strangulation, surgery, smoke inhalation, asphyxiation, drowning, choking, electrocution or drug or alcohol overdose), cardiac arrest, hypoglycemic neuronal damage, chemical dependencies and addictions (e.g., addictions to or dependencies on alcohol, opiates, benzodiazepines, nicotine, heroin or cocaine), ocular damage, retinopathy, retinal neuropathy, tinnitus, idiopathic and drug induced Parkinson's Disease, anxiety, panic disorder, schizophienia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting in a mammal, comprising administering to a mammal requiring such treatment a glutamate neurotransmission modulating effective amount of a compound of the formula I, or a pharmaceutically acceptable salt thereof.

This invention also relates to a pharmaceutical composition for treating a disorder or condition, the treatment of which can be effected or facilitated by modulating glutamate neurotransmission in a mammal, comprising a glutamate neurotransmission modulating effective amount of a compound of the formula I, or a pharmaceutically acceptable salt thereof, and a pharmaceutically acceptable carrier.

This invention also relates to compounds of formula I for use in treating a disorder or condition, the treatment of which can be effected or facilitated by modulating glutamate neurotransmission in a mammal, comprising administering to a mammal requiring such treatment a glutamate neurotransmission modulating effective amount of a compound of the formula I or a pharmaceutically acceptable salt thereof.

This invention also relates to compounds of formula I for use in treating a condition selected from stroke, cerebral ischemia, spinal cord trauma, head trauma, Alzheimer's Disease, Huntington's Chorea, amyotrophic lateral sclerosis, epilepsy, AIDS-induced dementia, muscular spasms, migraine headaches, urinary incontinence, psychosis, convulsions, perinatal hypoxia, hypoxia (such as conditions caused by strangulation, surgery, smoke inhalation, asphyxiation, drowning, choking, electrocution or drug or alcohol overdose), cardiac arrest, hypoglycemic neuronal damage, chemical dependencies and addictions (e.g., addictions to or dependencies on alcohol, opiates, benzodiazepines, nicotine, heroin or cocdine), ocular damage, retinopathy, retinal neuropathy, tinnitus, idiopathic and drug induced Parkinson's Disease, anxiety, panic disorder schizophienia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting in a mammal, comprising administering to a mammal in need of such treatment an amount of a compound of the formula I that is effective in treating such condition.

This invention also relates to a pharmaceutical composition for treating a condition selected from stroke, cerebral ischemia, spinal cord trauma, head trauma, Alzheimer's Disease, Huntington's Chorea, amyotrophic lateral sclerosis, epilepsy, AIDS-induced dementia, muscular spasms, migraine headaches, urinary incontinence, psychosis, convulsions, perinatal hypoxia, hypoxia (such as conditions caused by strangulation, surgery, smoke inhalation, asphyxiation, drowning, choking, electrocution or drug or alcohol overdose), cardiac arrest, hypoglycemic neuronal damage, chemical dependencies and addictions (e.g., addictions to or dependencies on alcohol, opiates, benzodiazepines, nicotine, heroin or cocaine), ocular damage, retinopathy, retinal neuropathy, tinnitus, idiopathic and drug induced Parkinson's Disease, anxiety, panic disorder, schizophienia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting in a mammal, comprising an amount of a compound of the formula I that is effective in treating such condition and a pharmaceutically acceptable carrier.

This invention also relates to compounds of formula I for use in treating a disorder or condition, the treatment of which can effected or facilitated by modulating glutamate neurotransmission in a mammal, comprising administering to a mammal requiring such treatment:
(a) a compound of the formula I, or a pharmaceutically acceptable salt thereof; and
(b) a serotonin reuptake inhibitor (e.g., sertraline, fluoxetine, fluvoxamine, etc.) or a serotonin-1A (5HT_{1A}) receptor ligand (e.g., buspirone, flesinoxan, etc.) or a pharmaceutically acceptable salt of such inhibitor or ligand;
wherein the amounts of the compound of formula I and the serotonin reuptake inhibitor or 5HT_{1A} receptor ligand that are employed in such method are such that the combination of the two active ingredients is effective in treating such disorder or condition.

This invention also relates to a pharmaceutical composition for treating a disorder or condition, the treatment of which can effected or facilitated by modulating glutamate neurotransmission in a mammal, comprising:
(a) a compound of the formula I, or a pharmaceutically acceptable salt thereof;
(b) a serotonin reuptake inhibitor (e.g., sertraline, fluoxetine, fluvoxamine, etc.) or a serotoin-1A (5HT_{1A}) receptor ligand (e.g., buspirone, flesinoxan, etc.) or a pharmaceutically acceptable salt of such inhibitor or ligand; and
(c) a pharmaceutically acceptable carrier;
wherein the amounts of the compound of formula I and the serotonin reuptake inhibitor or 5HT_{1A} receptor ligand that are contained in such compostion are such that the combination of the two active ingredients is effective in treating such disorder or condition.

This invention also relates to compounds of formula I for use in treating a disorder or condition, the treatment of which can effected or facilitated by modulating glutamate neurotransmission in a mammal, comprising administering to a mammal requiring such treatment:
(a) a glutamate neurotransmission modulating compound, or a pharmaceutically acceptable salt thereof; and
(b) a serotonin reuptake inhibitor (e.g., sertraline, fluoxetine, fluvoxamine, etc.) or a serotonin-1A (5HT_{1A}) receptor ligand (e.g., buspirone, flesinoxan, etc.) or a pharmaceutically acceptable salt of such inhibitor or ligand;
wherein the amounts of the glutamate neurotransmission modulating compound and the serotonin reuptake inhibitor or 5HT_{1A} receptor ligand that are employed in such method are such that the combination of the two active ingredients is effective in treating such disorder or condition.

This invention also relates to a pharmaceutical composition for treating a disorder or condition, the treatment of which can effected or facilitated by modulating glutamate neurotransmission in a mammal, comprising:
(a) a glutamate neurotransmission modulating compound or a pharmaceutically acceptable salt thereof;
(b) a serotonin reuptake inhibitor (e.g., sertraline, fluoxetine, fluvoxamine, etc.) or a serotoin-1A (5HT_{1A}) receptor ligand (e.g., buspirone, flesinoxan, etc.) or a pharmaceutically acceptable salt of such inhibitor or ligand; and
(c) a pharmaceutically acceptable carrier,
wherein the amounts of the glutamate neurotransmission modulating compound and the serotonin reuptake inhibitor or 5HT_{1A} receptor ligand that are contained in such compostion are such that the combination of the two active ingredients is effective in treating such disorder or condition.

This invention also relates to compounds of formula I for use in treating a disorder or condition, selected from stroke, cerebral ischemia, spinal cord trauma, head trauma, Alzheimer's Disease. Huntington's Chorea, amyotrophic lateral sclerosis, epilepsy, AIDS-induced dementia, muscular spasms, migraine headaches, urinary incontinence, psychosis, convulsions, perinatal hypoxia, hypoxia (such as conditions caused by strangulation, surgery, smoke inhalation, asphyxiation, drowning, choking, electrocution or drug or alcohol overdose), cardiac arrest, hypoglycemic neuronal damage, chemical dependencies and addictions (e.g., addictions to or dependencies on alcohol, opiates, benzodiazepines, nicotine, heroin or cocaine), ocular damage, retinopathy, retinal neuropathy, tinnitus, idiopathic and drug induced Parkinson's Disease, anxiety, panic disorder, schizophienia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting in a mammal, comprising administering to a mammal requiring such treatment:
(a) a compound of the formula **I,** or a pharmaceutically acceptable salt thereof; and
(b) a serotonin reuptake inhibitor (e.g., sertraline, fluoxetine, fluvoxamine, etc.) or a serotonin-1A (5HT_{1A}) receptor ligand (e.g., buspirone, flesinoxan, etc.) or a pharmaceutically acceptable salt of such inhibitor or ligand;
wherein the amounts of the compound of formula I and the serotonin reuptake inhibitor or 5HT_{1A} receptor ligand that are employed in such method are such that the combination of the two active ingredients is effective in treating such disorder or condition.

This invention also relates to a pharmaceutical composition for treating a disorder or condition selected from stroke, cerebral ischemia, spinal cord trauma, head trauma, Alzheimer's Disease, Huntington's Chorea, amyotrophic lateral sclerosis, epilepsy, AIDS-induced dementia, muscular spasms, migraine headaches, urinary incontinence, psychosis, convulsions, perinatal hypoxia, hypoxia (such as conditions caused by strangulation, surgery, smoke inhalation, asphyxiation, drowning, choking, electrocution or drug or alcohol overdose), cardiac arrest, hypoglycemic neuronal damage, chemical dependencies and addictions (e.g., addictions to or dependencies on alcohol, opiates, benzodiazepines, nicotine, heroin or cocaine), ocular damage, retinopathy, retinal neuropathy, tinnitus, idiopathic and drug induced Parkinson's Disease, anxiety, panic disorder, schizophienia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting in a mammal, comprising:
(a) a compound of the formula I, or a pharmaceutically acceptable salt thereof; and
(b) a serotonin reuptake inhibitor (e.g., sertraline, fluoxetine, fluvoxamine, etc.) or a serotoin-1A (5HT_{1A}) receptor ligand (e.g., buspirone, flesinoxan, etc.) or a pharmaceutically acceptable salt of such inhibitor or ligand; and
(c) a pharmaceutically acceptable carrier;
wherein the amounts of the compound of formula I and the serotonin reuptake inhibitor or 5HT_{1A} receptor ligand that are contained in such composition are such that the combination of the two active ingredients is effective in treating such disorder or condition.

Unless otherwise indicated, the alkyl groups referred to herein, as well as the alkyl moieties of other groups referred to herein (e.g., alkoxy), may be linear or branched, and they may also be cyclic (e.g., cyclopropyl, cyclobutyl, cyclopentyl, or cyclohexyl) or be linear or branched and contain cyclic moieties.

The term "treating" as used herein, refers to reversing, alleviating, inhibiting the progress of, or preventing the disorder or condition to which such term applies, or one or more symptoms of such disorder or condition. The term "treatment", as used herein, refers to the act of treating, as "treating" is defined immediately above.

Unless otherwise indicated, "halo" and "halogen", as used herein, refer to fluorine, bromine, chlorine or iodine.

Compounds of the formula I may have chiral centers and therefore may exist in different enantiomeric and diastereomic forms. This invention relates to all optical isomers and all other stereoisomers of compounds of the formula I, and to all racemic and other mixtures thereof, and to all pharmaceutical compositions and methods of treatment defined above that contain or employ such isomers or mixtures.

Formula I above includes compounds identical to those depicted but for the fact that one or more hydrogen or carbon atoms are replaced by isotopes thereof. Such compounds are useful as research and diagnostic tools in metabolism pharmokinetic studies and in binding assays. Specific applications in research include radioligand binding assays, autoradiography studies and in vivo binding studies.

### Detailed Description of the Invention

The compounds of formula I can be prepared according to the methods of Scheme 1. In the reaction Scheme and discussion that follow, unless otherwise indicated, n, Z, R¹, R², R³ R⁴, R⁵ and R⁶ and structural formulas I and II are defined as above.

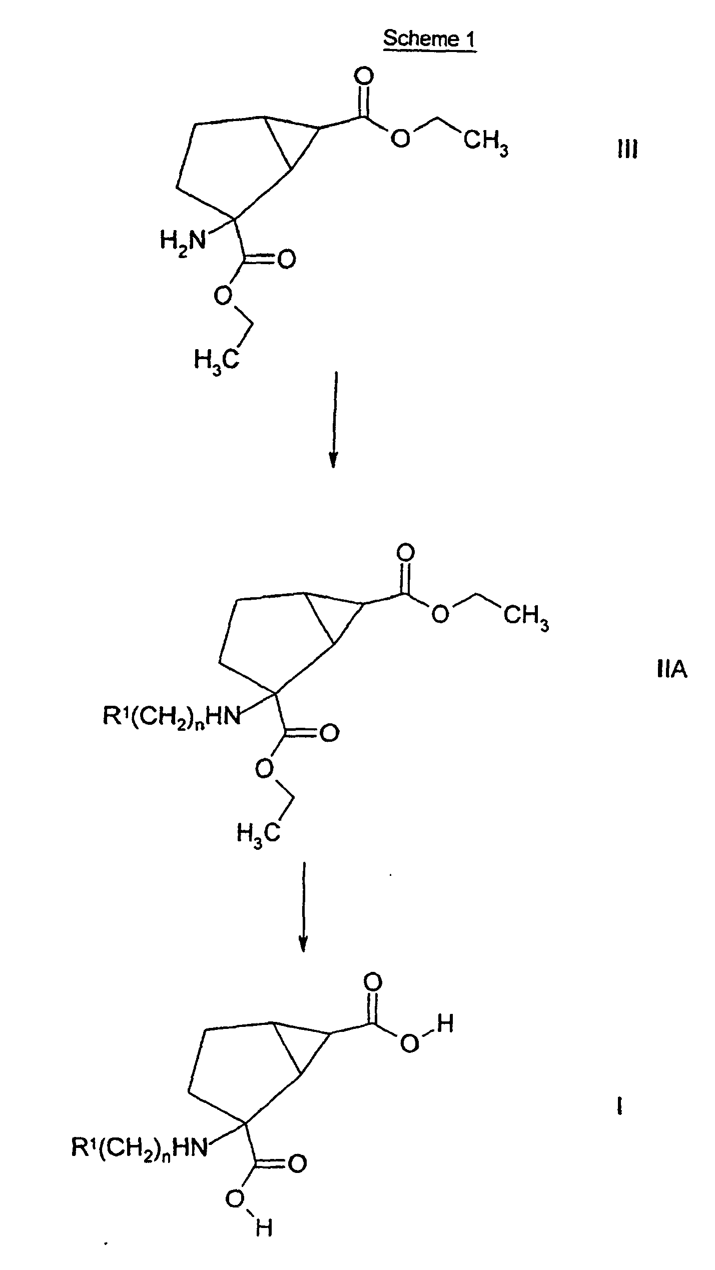

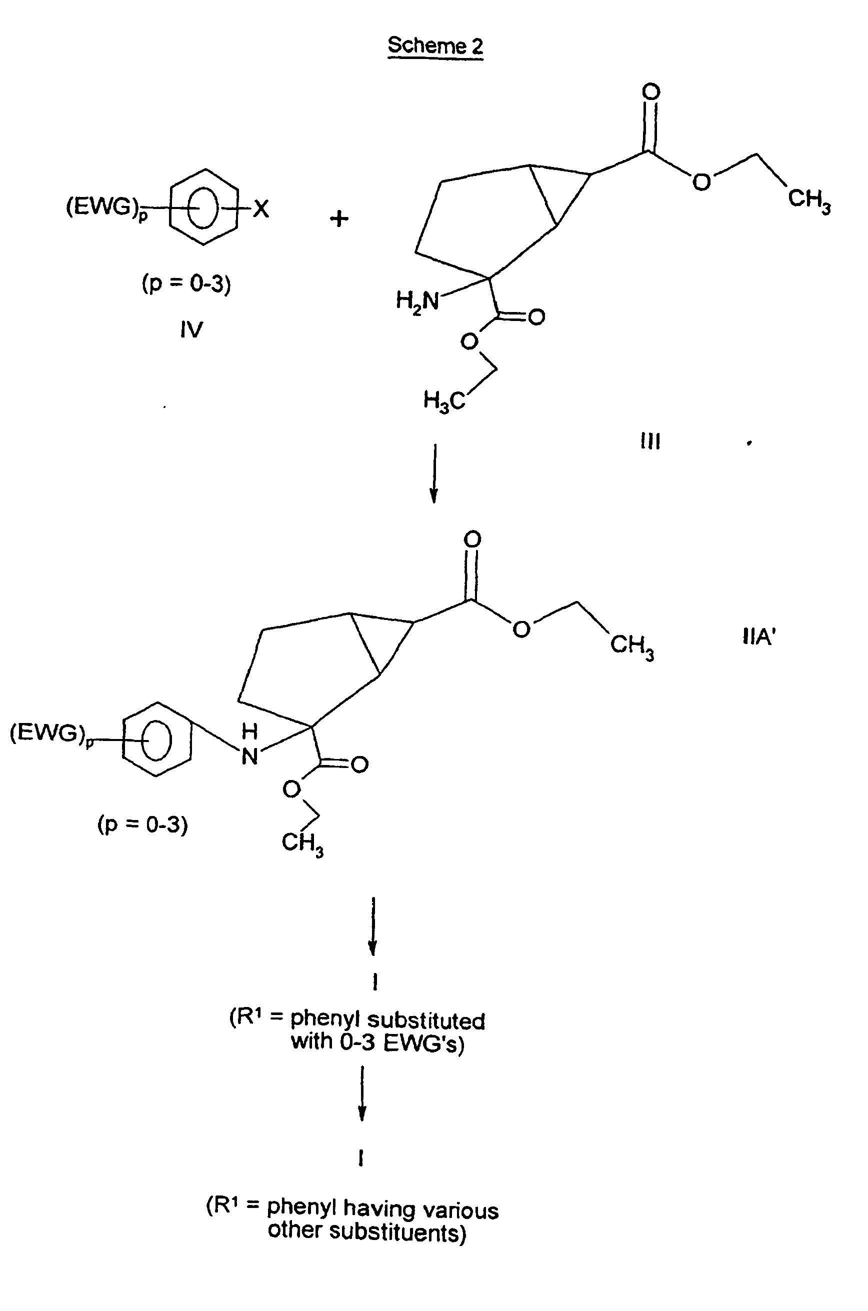

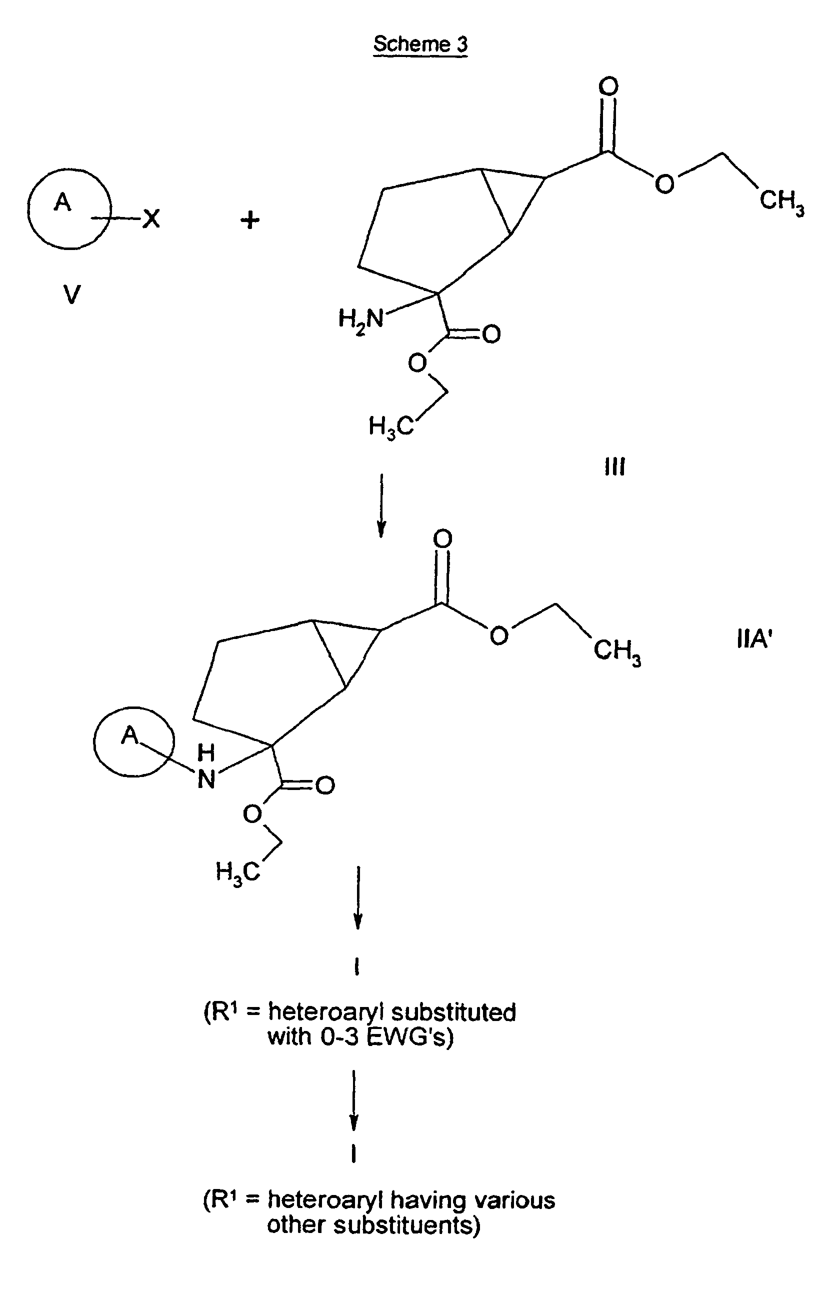

Scheme 1 illustrates the preparation of all compounds of the formula I in which there is an alkyl linkage of R¹ to the amino nitrogen of formula I.

Referring to Scheme I, a compound of the formula III is reacted with the appropriate aldehyde of the formula R¹(CH₂)ₘCHO, wherein m is equal to n-1, to form a compound of the formula IIA.

The above reductive amination reaction can be carried out using standard methods well known to those of skill in the art. This reaction is typically carried out in the presence of a reducing agent such as sodium cyanoborohydride, sodium triacetoxyborohydride, sodium borohydride, hydrogen (or a chemical hydrogen source such as formic acid or ammonium formate) and a metal catalyst such as platinum, palladium or rhodium, zinc and hydrochloric acid, borane dimethylsulfide or formic acid, at a temperature from about -60°C to about 50°C. Suitable reaction inert solvents for this reaction include lower alcohols (e.g., methanol, ethanol and isopropanol), dioxane, methylene chloride, dichloroethane, acetic acid and tetrahydrofuran (THF). Preferably, the solvent is methylene chloride or dichloroethane, the temperature is about 25°C, and the reducing agent is sodium triacetoxyborohydride.

The compounds of formula IIA formed in the above reaction can be converted onto the corresponding desired compounds of the formula I by subjecting them to acid or base hydrolysis, using methods well known to those of skill in the art. Suitable acids for the use in acid hydrolysis compound of the formula IIA include mineral acids such as hydrofluoric acid, sulfuric acid, hydrochloric acid and hydrobromic acid. Suitable bases for use in base hydrolysis of compounds of the formula IIA include alkali metal hydroxides and barium hydroxide. The reaction temperature for the acid and base hydrolysis reactions can range from about 0°C to about 100°C. Preferably, these reactions are carried out at about the reflux temperature of the reaction mixture.

Compounds of the formula I, wherein R¹ is aryl or heteroaryl and n is zero can be formed from the corresponding compounds of the formula IIA, as illustrated in Schemes 2 and 3, respectively. Compounds of the formula IIA wherein R¹ is aryl can be formed, as illustrated in Scheme 2, by reacting the corresponding compounds of the formula III, as depicted in Scheme 2, with a compound of the formula R¹X, wherein X is a leaving group such as halo, triflate, mesylate or tosylate. This reaction is generally carried out in a solvent such as ethanol, N,N-dimethylformamide (DMF), N,N-dimethylacetamide, acetonitrile, nitromethene, dioxane or dichloroethane, preferably DMF, at a temperature from about 0°C to about 160°C, preferably at about the reflux temperature. Scheme 2 specifically depicts the synthesis of compounds of the formula I wherein R¹ is an unsubstituted phenyl group or a phenyl group having from 0 to 3 electron withdrawing groups (EWG'S) as substituents. Examples of EWG's include, but are not limited to, nitro, (C₁-C₆)alkyl-SO-, (C₁-C₆)alkyl-SO₂-, (C₁-C₆)alkyl-O-C(=O)-, [(C₁₋C₆)alkyl)]₂NC(=O)-, cyano, -SO₂R⁴ and SO₂NR⁵R⁶.

In an analogous fashion, compounds of the formula I wherein R¹ is heteroaryl and n is zero may be prepared as illustrated in Scheme 3. Referring to Scheme 3, a compound of the formula III is reacted with a heteroaromatic compound of the formula V wherein ring A is a nitrogen containing heterocycle and X is a leaving group, as defined above, which is ortho to a ring nitrogen. The intermediate of the formula IIA that is formed in the foregoning reaction can be further hydrolyzed, under the conditions described above, to yeild the desired final product of formula I. Examples of compounds of the formula V are the following: 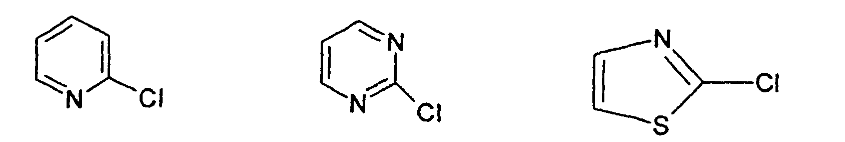 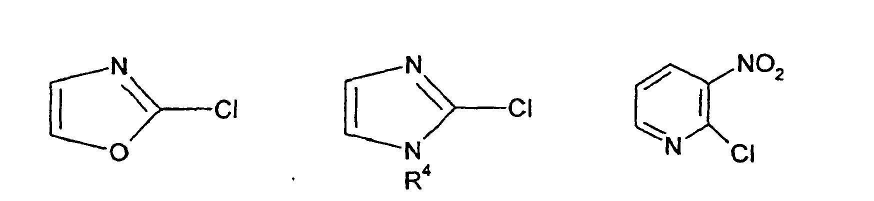 The presence on the above heteroaryl groups of additional EWG's further activates them.

Compounds wherein R¹ is another aryl or heteroaryl group can be synthesized in an analogous fashion starting with the appropriate compound of the formula R¹X.

Additional compounds of the formula I wherein R¹ is substituted aryl or heteroaryl may be obtained from compounds of the formula IIA wherein R¹ is a nitroaryl or nitroheteroraryl group, by employing well known synthetic chemical methods. For example, following procedures such as those described by Jerry March, Advanced Organic Chemistry, 4th edition pp721-725 and 1216-1217, the nitro group can be reduced to an amine. The newly formed amine can be replaced with other substituents by diazotization and further reaction as summarized in the above reference. For example, compounds of the formula I wherein R¹ is an aryl or heteroaryl group substituted with amino, mercapto, halo, cyano, or phenyl can be prepared in this manner.

The starting materials of formula IV and other compounds of the formula R¹X are either commerically available, known in the literature, or readily obtainable from commercially available or known compounds using methods that are known in the art.

The starting materials of the formula III are known in the literature. (See, e.g., European Patent Application 696577A1, referred to above).

Compounds of the formula 11 wherein one of R² and R³ is hydrogen can be prepared by partial hydrolysis of the corresponding compounds of formula IIA.

Unless indicated otherwise, the pressure of each of the above reactions is not critical. Generally, the reactions will be conducted at a pressure of about one to about three atmospheres, preferably at ambient pressure (about one atmosphere).

The preparation of other compounds of the formula I not specifically described in the foregoing experimental section can be accomplished using combinations of the reactions described above that will be apparent to those skilled in the art.

The compounds of the formula I that are basic in nature are capable of forming a wide variety of different salts with various inorganic and organic acids. The acid that can be used to prepare the pharmaceutically acceptable acid addition salts of the base compounds of this invention are those which form non-toxic acid addition salts, i.e., salts containing pharmacologically acceptable anions, such as hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [i.e., 1,1'-methylene-bis-(2-hydroxy-3-naphthoate)] salts. Although such salts must be pharmaceutically acceptable for administration to animals, it is often desirable in practice to initially isolate a compound of the formula I from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

Compounds of the formula that are acidic in nature are capable of forming base salts with various pharmacologically acceptable cations. These salts are all prepared by conventional techniques. The chemical bases that are used as reagents to prepare the pharmaceutically acceptable base salts of this invention are those which form non-toxic base salts with the acidic compounds of formula I. Such non-toxic base salts include those derived from such pharmacologically acceptable cations as sodium, potassium, calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The compounds of the formula I and the pharmaceutically acceptable salts thereof (hereinafter, also referred to, collectively, as "the active compounds of the invention") are useful for the treatment of neurodegenerative, psychotropic and drug or alcohol induced deficits and are potent metabotropic glutamate receptor ligands antagonists. The active compounds of the invention may therefore be used in the treatment or prevention of stroke, cerebral ischemia, spinal cord trauma, head trauma, Alzheimer's Disease, Huntington's Chorea, amyotrophic lateral sclerosis, epilepsy, AIDS-induced dementia, muscular spasms, migraine headaches, urinary incontinence, psychosis, convulsions, perinatal hypoxia, hypoxia (such as conditions caused by strangulation, surgery, smoke inhalation, asphyxiation, drowning, choking, electrocution or drug or alcohol overdose), cardiac arrest, hypoglycemic neuronal damage, chemical dependencies and addictions (e.g., addictions to or dependencies on alcohol, opiates, benzodiazepines, nicotine, heroin or cocaine), ocular damage, retinopathy, retinal neuropathy, tinnitus, idiopathic and drug induced Parkinson's Disease, anxiety, emesis, brain edema, chronic and acute pain, tardive dyskinesia and cerebral deficits subsequent to cardiac bypass surgery and grafting.

The following procedure can be used to determine the activity of the therapeutic agents of this invention as agonists and as antagonists of metabotropic glutamate receptors.

Chinese hamster ovary (CHO) cells were transfected with cDNA (mGluR2 and pcDNA3) using a calcium-phosphate method. Positive clones were selected for using geneticin (G418, Gibco, 500-700 µg/ml), and analyzed with RT-PCR for the presence of mGluR2 mRNA (primers for mGluR2: 5'-AAG TGC CCG GAG AAC TTC AAC GAA-3' AND 5'-AAA GCG ACG ACG TTG TTG AGT CCA-3'). Positive clones were grown to confluency and cAMP responses were measured in the presence of 10µM forskolin. Confluent clones were frozen and stored in liquid nitrogen.

Chinese hamster ovary (CHO) cells stably transfected with the rat metabotropic glutamate receptor, mGluR2, were grown to confluence in Dulbecco's Modified Eagle Medium (DMEM) (Gibco catalog # 11960-044), containing 10% dialysed fetal bovine serum, 1% proline, 0.11 mg/ml sodium pyruvate, 0.5 mg/ml Geneticin, 2 mM I-glutamine, and penicillin/streptomycin. Cells were harvested using a 5 mM ethylenediaminetetraacetic acid (EDTA) solution, and then spun down at 800 rpm in a 4°C refrigerated centrifuge. The remaining pellet was resuspended in a phosphate-buffered saline solution containing 30mM HEPES (Giboo, catino. 15630-080) 5 mM magnesium chloride (MgCl₂), 300 µM 3-Isobutyl-I-methylxanthine (IBMX), and 0.1% dextrose. The cell suspension was added in 200 µl aliquots to flat bottomed polypropylene tubes that were then placed in a 37°C heated water bath for 22 minutes. If a compound was being tested for antagonist activity, it was allowed to preincubate with the cells in the bath during the first 11 minutes. At the end of the 11 minutes, 5 µM forskolin plus a known concentration of an the test compound were added, and the incubation was continued for another 11 minutes. If a compound was being tested for agonist activity, the cells were allowed to shake in the bath for the initial 11 minutes, and then 5 µM forskolin plus a known concentration of agonist were added for the remaining 11 minute incubation. In either case, the reaction was stopped with 25 µl of 6N perchloric acid (PCA), and each tube was transferred immediately to an ice water bath. The pH of each sample was adjusted to approximately 8.0 with the addition of potassium hydroxide (KOH), and stabilized with the addition of Tris, pH 7.4. Aliquots (25 µl) were assayed in a commercial competitive binding kit (Amersham TRK.432). The samples were then harvested onto GF/B filters coated in 0.5% PEI using a 96-well Skatron harvester. Samples were quantified using a 1205 Betaplate liquid scintillation counter.

CPMs from the Betaplate reader were converted to pmoles cAMP/mg protein/minute of incubation with forskolin using an Excel spreadsheet. EC₅₀'s and IC₅₀'s can be calculated from linear regression of the concentration response data.

The following proceeding can be used to determine the agonist activity of the therapeutic agents of this invention as agonists of metabotropic glutamate receptors.

Chinese hamster ovary (CHO) cells stably transfected with the rat metabotropic glutamate receptor, mGluR2, were grown to confluence in DMEM (Gibco catalog # 11960-044), containing 10% dialyzed fetal bovine serum, 1% proline, 0.11 mg/mt sodium pyruvate, 0.5 mg/ml Geneticin, 2 mM I-glutamine, and penicillin/streptomycin. Cells are harvested using a 5 mM EDTA solution, and homogenized for 10 strokes with a glass-teflon hand held homogenizer, then 50 volumes of a phosphate buffered saline solution (PBS) are added and the solution is spun at 18,000 RPM for 10 minutes at 4°C. The pellet is rehomogenized and resuspended in assay buffer (100 mM HEPES, 1 mM EGTA, pH 7.5) at a concentration that will result in approx. 0.009 mg proteinlwell. A reaction mix containing 6mM MgCl₂, 0.5 mM adenosine triphosphate (ATP), 0.5 mM 3-isobutyl-1-methylxanthine (IBMX), 0.1 mM guanosine triphosphate (GTP), 10 mM phosphocreatine, 0.31 mg/ml creatine phosphokinase (final concentrations in assay) is prepared just prior to the initiation of the experiment. 20 µl of test compound, 20 µl of forskolin (5 µM final), 20 µl of reaction mix, and 40 µl of tissue are added in consecutive order to a 96-well polypropaline plate. The plate is incubated at 37°C in a heated water bath for 15 minutes. The reaction is stopped with the addition of 50 µl of 40 mM EDTA. The plate is then transferred to ice and shaken for 10-15 minutes before a 25 µl aliquot is removed for analysis in a commercial competitive binding kit (Amersham TRK.432). After a 2-18 hour incubation in the refrigerator, the samples are harvested onto GF/B filters coated in 0.5% polyethylenimine (PEI) using a 96-well Stcatron harvester. Samples were quantified using a 1205 Betaptate liquid scintillation counter.

CPMs from the Betaplate reader are converted to pmoles cAMP/well using an Excel spreadsheet. Agonist compounds are identified by percent reduction of the forskolin signal, also in Excel. EC₅₀'s are calculated from linear regression of the concentration response data.

The compositions of the present invention may be formulated in a conventional manner using one or more pharmaceutically acceptable carriers. Thus, the active compounds of the invention may be formulated for oral, buccal, transdermal (e.g., patch), intranasal, parenteral (e.g., intravenous, intramuscular or subcutaneous) or rectal administration or in a form suitable for administration by inhalation or insufflation.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium phosphate); lubricants (e.g., magnesium stearate, talc or silica); disintegrants (e.g., potato starch or sodium starch glycollate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., almond oil, oily esters or ethyl alcohol); and preservatives (e.g., methyl or propyl p-hydroxybenzoates or sorbic acid).

For buccal administration the composition may take the form of tablets or lozenges formulated in conventional manner.

The active compounds of the invention may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, e.g., sterile pyrogen-free water, before use.

The active compounds of the invention may also be formulated in rectal compositions such as suppositories or retention enemas, e.g., containing conventional suppository bases such as cocoa butter or other glycerides.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

A proposed dose of the active compounds of the invention for oral, parenteral or buccal administration to the average adult human for the treatment of the conditions referred to above (e.g., stroke) is 0.01 to 50 mglkg of the active ingredient per unit dose which could be administered, for example, 1 to 4 times per day.

Aerosol formulations for treatment of the conditions referred to above (e.g., stroke) in the average adult human are preferably arranged so that each metered dose or "puff" of aerosol contains 20mg to 1000mg of the compound of the invention. The overall daily dose with an aerosol will be within the range 100 mg to 10 mg. Administration may be several times daily, for example 2, 3, 4 or 8 times, giving for example, 1, 2 or 3 doses each time.

The following Examples illustrate the preparation of the compounds of the present invention. Commercial reagents were utilized without further purification. Melting points are uncorrected. All NMR data were recorded at 250, 300 or 400 MHz in deuterochloroform unless otherwise specified and are reported in parts per million (δ) and are referenced to the deuterium lock signal from the sample solvent. All non-aqueous reactions were carried out in dry glassware with dry solvents under an inert atmosphere for convenience and to maximize yields. All reactions were stirred with a magnetic stirring bar unless otherwise stated. Unless otherwise stated, all mass spectra were obtained using chemical impact conditions. Ambient or room temperature refers to 20-25°C. Melting points are uncorrected. HPLC conditions are the following.
(a) Column: Waters Symmetry C18 3.9x150mm, part no. WAT046980, lot no.T71961R 87
   Solvent System: Isocratic 75% 5mM Ammonium acetate buffer unadjusted/25% Acetonitrile
   Flow Rate: 0.8mUmin.
   Run Time: 10min.
   Injection Volume: 20µL
   Detection Wavelength: 206.4nm
b) Column: Waters Symmetry C18 4.6x250mm, part no. WAT054275, lot no. T71141U 02
   Solvent System: Isocratic 5% 5mM Ammonium acetate buffer unadjusted/ 95% Acetonitrile
   Flow Rate: 0.8mL/min.
   Run Time: 10min.
   Injection Volume: 20µL
   Detection Wavelength: 203.9nm
c) Column: Waters Spherisorb Phenyl 4.6x250mm
   Solvent System: Isocratic
   Flow Rate: 1.0mL/min.
   Run Time: 10min.
   Injection Volume: 15µL
   Detection Wavelength: 203.9nm

### EXAMPLE 1

A. Sodium triacetoxyborohydride (0.0403 g, 0.19 mmol) was added to a mixture of diethyl 2-aminobicyclo[3.1.0]hexane-2,6-dicarboxylate (0.0092 g, 0.038 mmol) and 0.046 mmol of benzaldehyde in dichloromethane (0.92 mL) in a 1.0 mL screw cap vial and the mixture was agitated at ambient temperature overnight. Activity 1 alumina B (50-200 mesh, 0.10 g) was added and the mixture was agitated another 30 min before filtering through activity 1 alumina B (0.5 g). The alumina was washed with 2 mL of 5% methanol I dichloromethane. An aliquot of the filtrate was analyzed by HPLC for unreacted diethyl 2-aminobicyclo[3.1.0]hexane-2,6-dicarboxylate. If this starting material was present, the mixture was not carried on to step 2. The solution was concentrated at 50°C (19 inches Hg) to give an oily intermediate product.
B. The intermediate product from step 1 was combined with 6 N HCl (0.50 mL) in a 1 mL screw cap vial and agitated at 100°C overnight. An aliquot was extracted for HPLC analysis. If HPLC analysis indicated that the intermediate product from step 1 was consumed, the reaction was cooled to ambient temperature and applied to strong cation exchange resin (Burdick and Jackson SCX, 0.50 g which had previously been washed with water until the eluant pH was ~4.5). The product was eluted with 2 mL water. Concentration of the eluant at 70°C (19 inches Hg) gave the off white solid product. The product was characterized by HPLC retention time and mass spectrum parent ion.

The following compounds of the formula I were prepared using an analogous procedure.

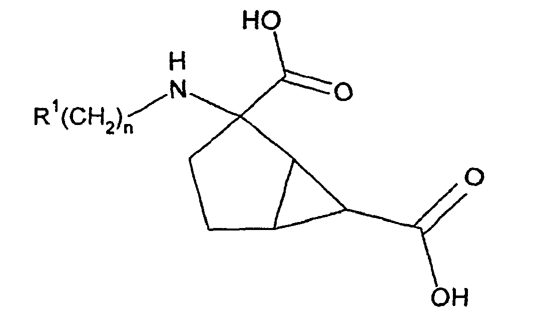

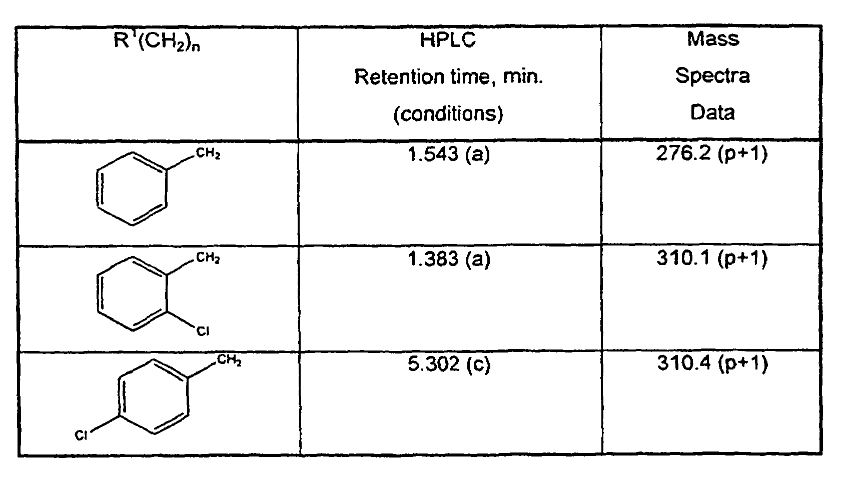

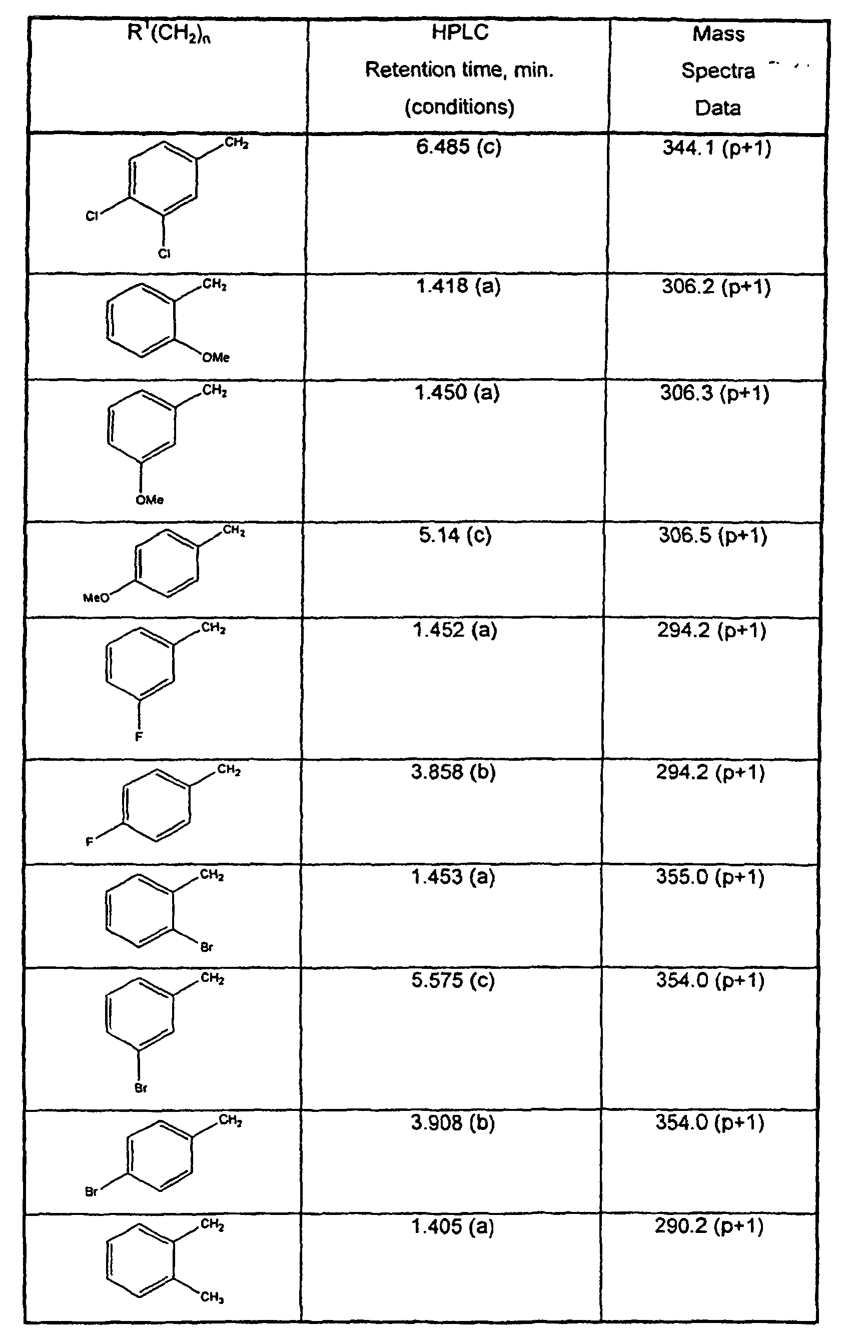

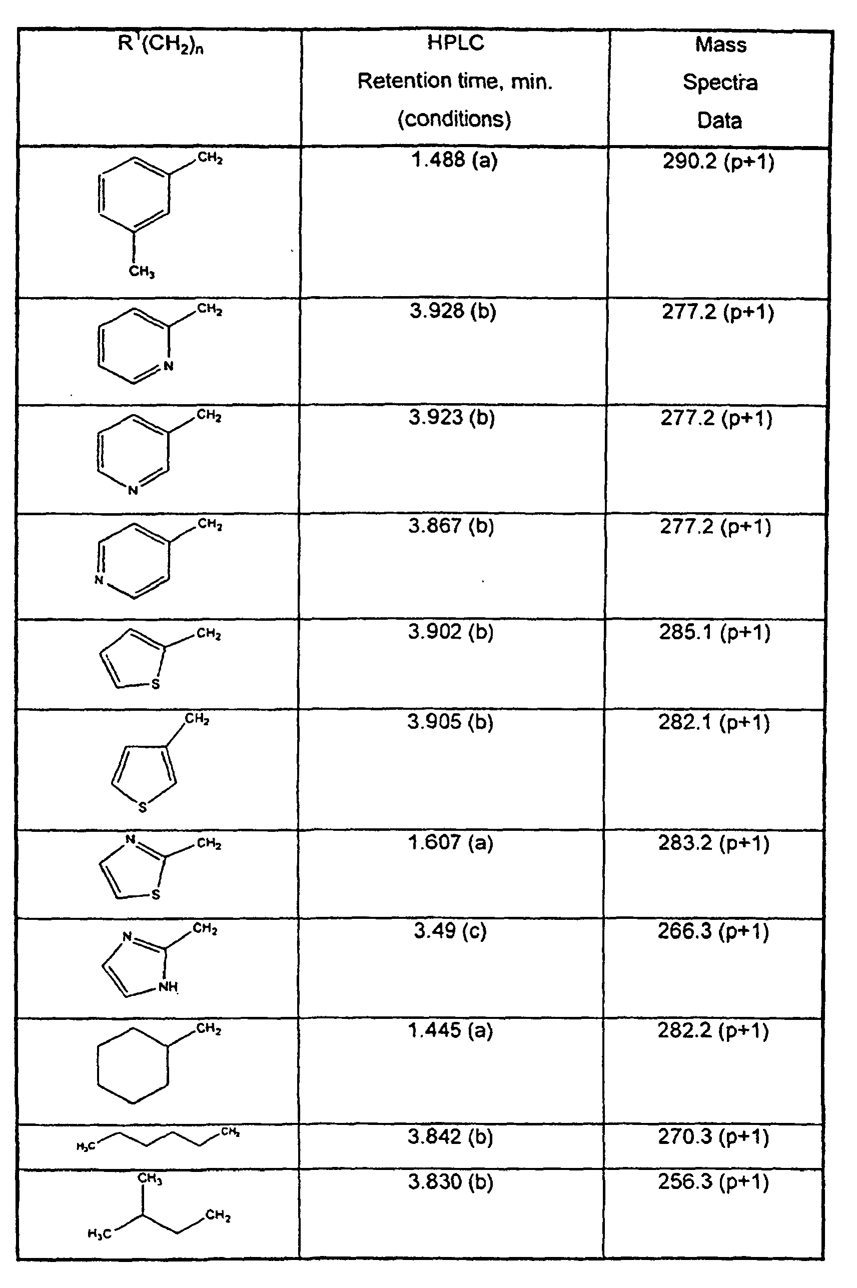

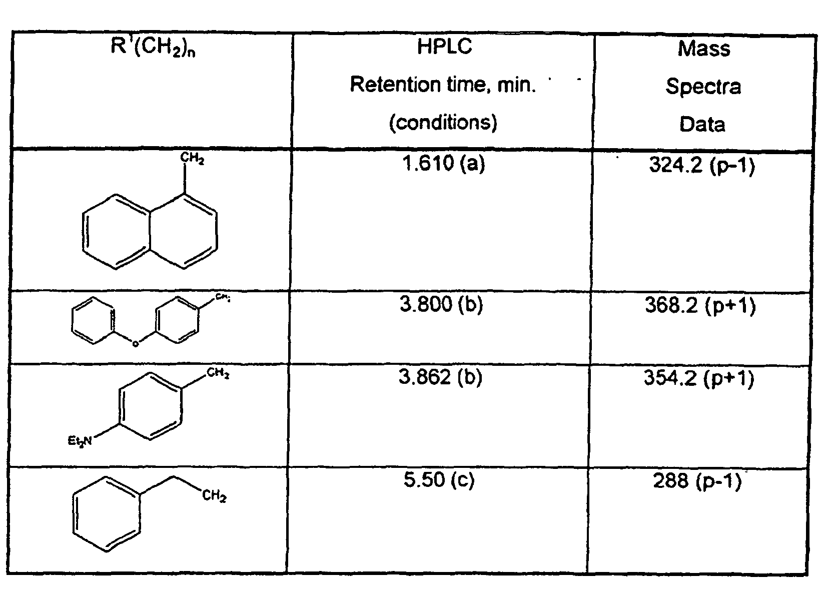

## Claims

1. A compound of the formula 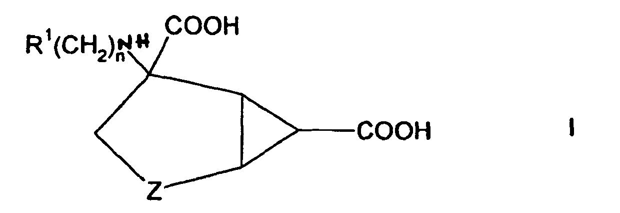 wherein n is 0-6;
Z is (C₁-C₄) alkylene, oxygen, sulfur, NH or N(C₁-C₆)alkyl;
R¹ is hydrogen, (C₁-C₆)alkyl, aryl or heteroaryl, wherein aryl is selected from phenyl and naphthyl and said heteroaryl is selected from 5 and 6 membered aromatic heterocyclic rings that contain from one to four heteroatoms selected, independently, from nitrogen, oxygen and sulfur, and wherein said aryl and heteraryl moieties can optionally be substituted with one or more substituents, preferably with one or two substituents, that are selected, independently, from halo (e.g., fluoro, chloro, bromo or iodo), -SO(C₁-C₆)alkyl, -SO₂R⁴, -SO₂NR⁵R⁶, (C₁-C₆)alkyl optionally substituted with from one to seven fluorine atoms, (C₁₋C₆)alkoxy optionally substituted with from one to seven fluorine atoms, amino, nitro, cyano, carboxy, -CO₂(C₁-C₆)alkyl, (C₁-C₆)alkylamino, di-[(C₁-C₆)alkyl]amino phenoxy, anilino and phenylthio;
R⁴ is -O(C₁ - C₆)alkyl, phenyl or (C₁ - C₆)alkyl; and
R⁵ and R⁶ are selected, independently, from hydrogen, (C₁ - C₆)alkyl and phenyl;
with the proviso that R¹ can not be hydrogen when n is zero, and with the proviso that none of the foregoing heteroaryl moieties may contain more than one ring oxygen atom or more than one ring sulfur atom;
or a pharmaceutically acceptable salt thereof.

2. A compound according to claim 1 wherein Z is CH₂.

3. A compound according to claim 1 or claim 2 wherein R¹ is a disubstituted phenyl group having substituents at the meta and para positions.

4. A compound according to claim 1, 2 or 3 wherein R² is a monosubstituted or disubstituted phenyl group wherein one substituent is in the para position.

5. A compound according to claim 1 or claim 2 wherein R¹(CH₂)ₙ is one of the following groups:

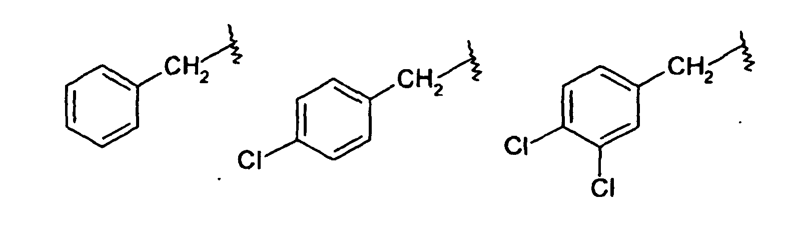

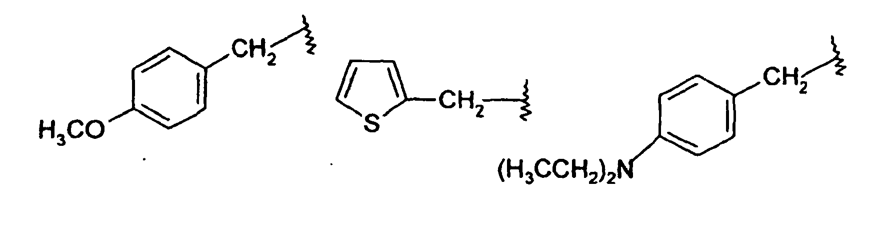

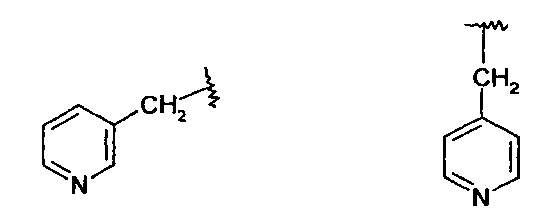

6. A pharmaceutical composition for treating: (a) a disorder or condition that can be treated by modulating glutamate neurotransmission in a mammal; or (b) a disorder or condition selected from stroke, cerebral ischemia, spinal cord trauma, head trauma, Alzheimer's Disease, Huntington's Chorea, amyotrophic lateral sclerosis, AIDS-induced dementia, muscular spasms, migraine headaches, urinary incontinence, psychosis, convulsions, perinatal hypoxia, hypoxia, cardiac arrest, hypoglycemic neuronal damage, chemical dependencies and addictions, ocular damage and retinopathy, idiopathic and drug induced Parkinson's Disease, anxiety, panic disorder, schizophrenia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting in a mammal, comprising an amount of a compound according to claim 1 that is effective in treating such disorder or condition and a pharmaceutically acceptable carrier.

7. The use of a compound or salt according to claim 1 in the manufacture of a medicament for treating: (a) a disorder or condition that can be treated by modulating glutamate neurotransmission in a mammal; or (b) a disorder or condition selected from stroke, cerebral ischemia, spinal cord trauma, head trauma, Alzheimer's Disease, Huntington's Chorea, amyotrophic lateral sclerosis, AIDS-induced dementia, muscular spasms, migraine headaches, urinary incontinence, psychosis, convulsions, perinatal hypoxia, hypoxia, cardiac arrest, hypoglycemic neuronal damage, opiate tolerance and withdrawal, ocular damage and retinopathy, idiopathic and drug induced Parkinson's Disease, anxiety, panic disorder, schizophrenia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting.

8. A pharmaceutical composition for treating: (a) a disorder or condition that can be treated by mediating glutamate neurotransmission in a mammal; or (b) a disorder or condition selected from stroke, cerebral ischemia, spinal cord trauma, head trauma, Alzheimer's Disease, Huntington's Chorea, amyotrophic lateral sclerosis, AIDS-induced dementia, muscular spasms, migraine headaches, urinary incontinence, psychosis, convulsions, perinatal hypoxia, hypoxia, cardiac arrest, hypoglycemic neuronal damage, chemical dependencies and addictions, ocular damage and retinopathy, idiopathic and drug induced Parkinson's Disease, anxiety, panic disorder, schizophrenia, depression, bipolar disorder, obsessive-compulsive disorder, Tourette's syndrome, emesis, brain edema, chronic and acute pain, tardive dyskinesia and cerebral deficits subsequent to cardiac bypass surgery and grafting in a mammal, comprising:
(a) a compound according to claim 1 or a pharmaceutically acceptable salt thereof;
(b) a serotonin reuptake inhibitor or a 5HT_{1A} receptor ligand, or a pharmaceutically acceptable salt of such inhibitor or ligand; and
(c) a pharmaceutically acceptable carrier;
wherein the compound according to claim 1 and the 5HT_{1A} receptor ligand or serotonin reuptake inhibitor that are contained in such composition are present in amounts such that the combination of the two active ingredients is effective in treating such disorder or condition.

9. The use of (i) a compound or salt according to claim 1, and (ii) a serotonin reuptake inhibitor or a 5HT1a ligand or a pharmaceutically acceptable salt of such inhibitor or ligand, in the manufacture of a medicament for treating (a) a disorder or condition that can be treated by modulating glutamate neurotransmission in a mammal: or (b) a disorder or condition selected from stroke, cerebral ischemia, spinal cord trauma, head trauma, Alzheimer's Disease, Huntington's Chorea, amyotrophic lateral sclerosis, AIDS-induced dementia, muscular spasms, migraine headaches, urinary incontinence, psychosis, convulsions, perinatal hypoxia, hypoxia, cardiac arrest, hypoglycemic neuronal damage, opiate tolerance and withdrawal, ocular damage and retinopathy, idiopathic and drug induced Parkinson's Disease, anxiety, panic disorder, schizophrenia, depression, bipolar disorder, obsessive-compulsive disorder. Tourette's syndrome, emesis, brain edema, chronic and acute pain, tardive dyskinesia and cerebral deficits subsequent to cardiac bypass surgery and grafting.

10. A pharmaceutical composition comprising a compound or salt according to any one of claims 1 to 6 and a pharmaceutically acceptable carrier.

11. A compound or salt according to any one of claims 1 to 6 for use in medicine.

## Patentansprüche

1. Verbindung der Formel 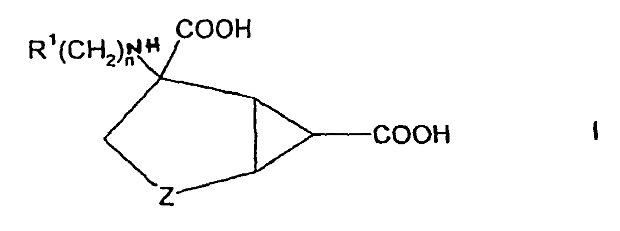 worin n 0-6 ist;
Z (C₁-C₄)Alkylen, Sauerstoff, Schwefel, NH oder N(C₁₋C₆)Alkyl ist;
R¹ Wasserstoff, (C₁-C₆)Alkyl, Aryl oder Heteroaryl ist, wobei Aryl aus Phenyl und Naphthyl ausgewählt ist und das Heteroaryl aus 5- und 6-gliedrigen aromatischen heterocyclischen Ringen, die ein bis vier Heteroatome, unabhängig ausgewählt aus Stickstoff, Sauerstoff und Schwefel, enthalten, und wobei die Aryl- und Heteroarylgruppierungen gegebenenfalls mit einem oder mehreren Substituenten, vorzugsweise mit einem oder zwei Substituenten, substituiert sein können, die unabhängig aus Halogen (z.B. Fluor, Chlor, Brom oder Iod), -SO (C₁-C₆) Alkyl, -SO₂R⁴, -SO₂NR⁵R⁶, (C₁-C₆)Alkyl, gegebenenfalls substituiert mit ein bis sieben Fluoratomen, (C₁-C₆)Alkoxy, gegebenenfalls substituiert mit ein bis sieben Fluoratomen, Amino, Nitro, Cyano, Carboxy, -CO₂(C₁-C₆)Alkyl, (C₁-C₆)Alkylamino, Di-[(C₁-C₆)alkyl]aminophenoxy, Anilino und Phenylthio;
R⁴ -O(C₁-C₆)Alkyl, Phenyl oder (C₁-C₆)Alkyl ist und
R⁵ und R⁶ unabhängig aus Wasserstoff, (C₁-C₆)Alkyl und Phenyl ausgewählt sind;
mit der Maßgabe, dass R¹ nicht Wasserstoff sein kann, wenn n Null ist, und mit der Maßgabe, dass keine der vorstehenden Heteroarylgruppierungen mehr als ein Ringsauerstoffatom oder mehr als ein Ringschwefelatom enthalten kann;
oder ein pharmazeutisch annehmbares Salz davon.

2. Verbindung nach Anspruch 1, wobei Z CH₂ ist.

3. Verbindung nach Anspruch 1 oder Anspruch 2, wobei R¹ eine disubstituierte Phenylgruppe ist, die Substituenten in den meta- und para-Positionen hat.

4. Verbindung nach Anspruch 1, 2 oder 3, wobei R² eine monosubstituierte oder disubstituierte Phenylgruppe, in der ein Substituent in der para-Position ist, ist.

5. Verbindung nach Anspruch 1 oder Anspruch 2, wobei R¹(CH₂)ₙ eine der folgenden Gruppen ist:

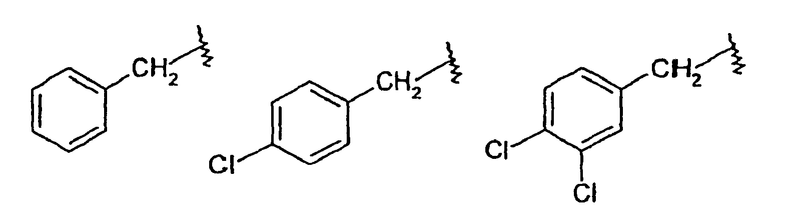

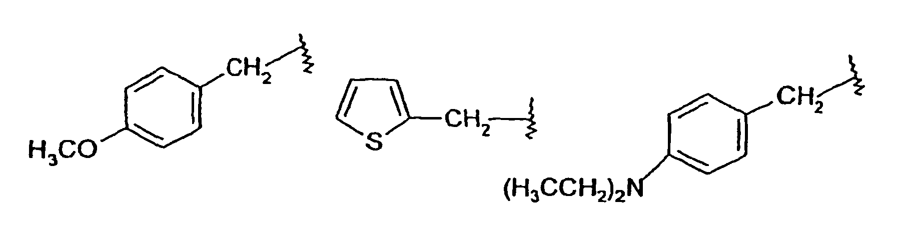

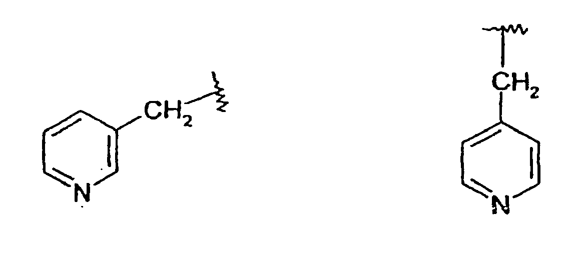

6. Pharmazeutische Zusammensetzung zur Behandlung: (a) einer Störung oder eines Zustandes, die/der durch Modulieren der Glutamatneurotransmission bei einem Säuger behandelt werden kann; oder (b) einer Störung oder eines Zustandes, ausgewählt aus Schlaganfall, zerebraler Ischämie, Rückenmarktrauma, Kopftrauma, Alzheimer'scher Krankheit, Huntington-Chorea, amytropher Lateralsklerose, AIDS-induzierter Demenz, Muskelspasmus, Migräne, Harninkontinenz, Psychose, Konvulsionen, Perinatalhypoxie, Hypoxie, Herzstillstand, hypoglykämischer Nervenschädigung, Abhängigkeiten von Chemikalien und Suchten, Augenschädigung und Retinopathie, ideopathischer und Arzneimittel- bzw. Drogen-induzierter Parkinson'scher Krankheit, Angst, Panikstörung, Schizophrenie, Depression, bipolarer Störung, Zwangsneurose, Tourette-Syndrom, Emesis, Hirnödem, chronischen und akuten Schmerzen, Spätdyskinesie und zerebralen Defiziten nach Herz-Bypass-Operation und Transplantation bei einem Säuger, die eine Menge einer Verbindung nach Anspruch 1, die bei der Behandlung einer solchen Störung oder eines solchen Zustands wirksam ist, und einen pharmazeutisch annehmbaren Träger umfasst.

7. Verwendung einer Verbindung oder eines Salzes nach Anspruch 1, bei der Herstellung eines Medikaments zur Behandlung: (a) einer Störung oder eines Zustands, die/der durch Modulieren der Glutamatneurotransmission bei einem Säuger behandelt werden kann; oder (b) einer Störung oder eines Zustands, ausgewählt aus Schlaganfall, zerebraler Ischämie, Rückenmarktrauma, Kopftrauma, Alzheimer'scher Krankheit, Huntington-Chorea, amytropher Lateralsklerose, AIDS-induzierter Demenz, Muskelspasmus, Migräne, Harninkontinenz, Psychose, Konvulsionen, Perinatalhypoxie, Hypoxie, Herzstillstand, hypoglykämischer Nervenschädigung, Opiat-Toleranz und Entzugserscheinungen, Augenschädigung und Retinopathie, ideopathischer und Arzneimittel- bzw. Drogen-induzierter Parkinson'scher Krankheit, Angst, Panikstörung, Schizophrenie, Depression, bipolarer Störung, Zwangsneurose, Tourette-Syndrom, Emesis, Hirnödem, chronischen und akuten Schmerzen, Spätdyskinesie und zerebralen Defiziten nach Herz-Bypass-Operation und Transplantation.

8. Pharmazeutische Zusammensetzung zur Behandlung: (a) einer Störung oder eines Zustands, die/der durch Modulieren der Glutamatneurotransmission bei einem Säuger behandelt werden kann; oder (b) einer Störung oder eines Zustandes, ausgewählt aus Schlaganfall, zerebraler Ischämie, Rückenmarktrauma, Kopftrauma, Alzheimer'scher Krankheit, Huntington-Chorea, amytropher Lateralsklerose, AIDS-induzierter Demenz, Muskelspasmus, Migräne, Harninkontinenz, Psychose, Konvulsionen, Perinatalhypoxie, Hypoxie, Herzstillstand, hypoglykämischer Nervenschädigung, Abhängigkeiten von Chemikalien und Suchten, Augenschädigung und Retinopathie, ideopathischer und Arzneimittel- bzw. Drogen-induzierter Parkinson'scher Krankheit, Angst, Panikstörung, Schizophrenie, Depression, bipolarer Störung, Zwangsneurose, Tourette-Syndrom, Emesis, Hirnödem, chronischen und akuten Schmerzen, Spätdyskinesie und zerebralen Defiziten nach Herz-Bypass-Operation und Transplantation bei einem Säuger, umfassend:
(a) eine Verbindung nach Anspruch 1 oder ein pharmazeutisch annehmbares Salz davon;
(b) einen Serotonin-Wiederaufnahme-Inhibitor oder einen 5HT_{1A}-Rezeptorliganden oder ein pharmazeutisch annehmbares Salz eines solchen Inhibitors oder Liganden und
(c) einen pharmazeutisch annehmbaren Träger;
wobei die Verbindung nach Anspruch 1 und der 5HT_{1A-}Rezeptorligand oder Serotonin-Wiederaufnahme-Inhibitor, die in einer solchen Zusammensetzung enthalten sind, in Mengen vorliegen, dass die Kombination der zwei aktiven Ingredienzien bei der Behandlung einer solchen Störung oder eines solchen Zustands wirksam ist.

9. Verwendung von (i) einer Verbindung oder eines Salzes nach Anspruch 1 und (ii) eines Serotonin-Wiederaufnahme-Inhibitors oder eines 5HT₁ₐ-Liganden oder eines pharmazeutisch annehmbaren Salzes eines solchen Inhibitors oder Liganden bei der Herstellung eines Medikaments zur Behandlung (a) einer Störung oder eines Zustands, die/der durch Modulieren der Glutamatneurotransmission bei einem Säuger behandelt werden kann; oder (b) einer Störung oder eines Zustandes, ausgewählt aus Schlaganfall, zerebraler Ischämie, Rückenmarktrauma, Kopftrauma, Alzheimer'scher Krankheit, Huntington-Chorea, amytropher Lateralsklerose, AIDS-induzierter Demenz, Muskelspasmus, Migräne, Harninkontinenz, Psychose, Konvulsionen, Perinatalhypoxie, Hypoxie, Herzstillstand, hypoglykämischer Nervenschädigung, Opiat-Toleranz und Entzugserscheinungen, Augenschädigung und Retinopathie, ideopathischer und Arzneimittel- bzw. Drogen-induzierter Parkinson'scher Krankheit, Angst, Panikstörung, Schizophrenie, Depression, bipolarer Störung, Zwangsneurose, Tourette-Syndrom, Emesis, Hirnödem, chronischen und akuten Schmerzen, Spätdyskinesie und zerebralen Defiziten nach Herz-Bypass-Operation und Transplantation.

10. Pharmazeutische Zusammensetzung, die eine Verbindung oder ein Salz nach einem der Ansprüche 1 bis 6 und einen pharmazeutisch annehmbaren Träger umfasst.

11. Verbindung oder Salz nach einem der Ansprüche 1 bis 6 zur Verwendung in Medizin.

## Revendications

1. Composé de formule 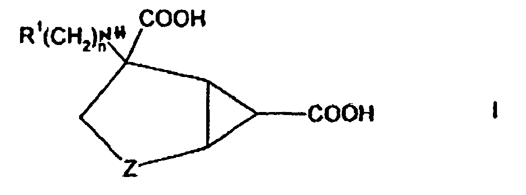 dans laquelle n a une valeur de 0 à 6 ;
Z représente un groupe alkylène en C₁ à C₄, un atome d'oxygène, un atome de soufre, un groupe NH ou un groupe N(alkyle en C₁ à C₆) ;
R¹ représente un atome d'hydrogène, un groupe alkyle en C₁ à C₆, aryle ou hétéroaryle, le groupe aryle étant choisi entre les groupes phényle et naphtyle et ledit groupe hétéroaryle étant choisi parmi des noyaux hétérocycliques aromatiques penta- et hexagonaux qui contiennent 1 à 4 hétéroatomes choisis, indépendamment, entre l'azote, l'oxygène et le soufre, et lesdits groupements aryle et hétéroaryle pouvant être facultativement substitués avec un ou plusieurs substituants, de préférence avec un ou deux substituants, qui sont choisis, indépendamment, entre des substituants halogéno (par exemple fluoro, chloro, bromo ou iodo), -SO(alkyle en C₁ à C₆), -SO₂R⁴, -SO₂NR⁵R⁶, alkyle en C₁ à C₆ facultativement substitué avec un à sept atomes de fluor, alkoxy en C₁ à C₆ facultativement substitué avec un à sept atomes de fluor, amino, nitro, cyano, carboxy, -CO₂(alkyle en C₁ à C₆), alkylamino en C₁ à C₆, di (alkyle en C₁ à C₆)amino phénoxy, anilino et phénylthio ;
R⁴ représente un groupe -O(alkyle en C₁ à C₆), phényle ou alkyle en C₁ à C₆ ; et
R⁵ et R⁶ sont choisis, indépendamment, entre l'hydrogène, un groupe alkyle en C₁ à C₆ et un groupe phényle ;
sous réserve que R¹ ne puisse représenter l'hydrogène lorsque n est égal à zéro, et sous réserve qu'aucun des groupements hétéroaryle précités ne puisse contenir plus d'un atome d'oxygène dans le noyau ou plus d'un atome de soufre dans le noyau ;
ou un de ses sels pharmaceutiquement acceptables.

2. Composé suivant la revendication 1, dans lequel Z représente un groupe CH₂.

3. Composé suivant la revendication 1 ou la revendication 2, dans lequel R¹ représente un groupe phényle disubstitué portant des substituants en positions méta et para.

4. Composé suivant la revendication 1, 2 ou 3, dans lequel R² représente un groupe phényle monosubstitué ou disubstitué, dans lequel un substituant est en position para.

5. Composé suivant la revendication 1 ou la revendication 2, dans lequel R¹(CH₂)ₙ est un des groupes suivants :

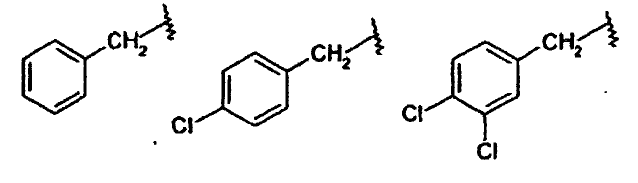

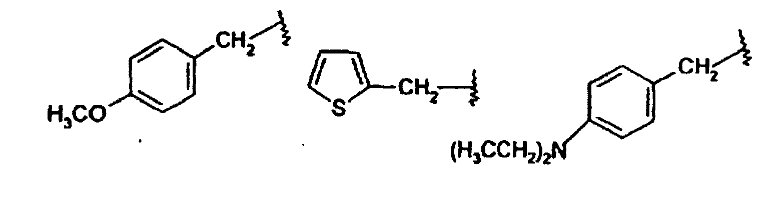

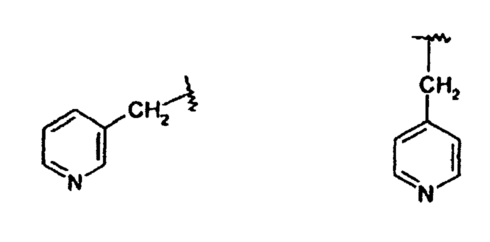

6. Composition pharmaceutique pour le traitement : (a) d'une affection ou d'un trouble qui peut être traité en modulant la neurotransmission du glutamate chez un mammifère ; ou (b) d'une affection ou d'un trouble choisi entre un ictus, une ischémie cérébrale, un traumatisme de la moelle épinière, un traumatisme crânien, la maladie d'Alzheimer, la chorée de Huntington, la sclérose latérale amyotrophique, la démence induite par le SIDA, les spasmes musculaires, les céphalées de la migraine, l'incontinence urinaire, la psychose, les convulsions, l'hypoxie périnatale, une hypoxie, un arrêt cardiaque, l'altération neuronale hypoglycémique, des dépendances et accoutumances chimiques, l'altération oculaire et la rétinopathie, la maladie de Parkinson idiopathique et la maladie de Parkinson induite par un médicament, l'anxiété et un trouble panique, la schizophrénie, la dépression, un trouble bipolaire, le syndrome obsessionnel compulsif, le syndrome de Tourette, les vomissements, un oedème cérébral, la douleur chronique, et la douleur aiguë, la dyskinésie tardive et les déficiences cérébrales faisant suite à une intervention chirurgicale de dérivation cardiaque et une greffe cardiaque chez un mammifère, comprenant une quantité d'un composé suivant la revendication 1 qui est efficace dans le traitement de cette affection ou de ce trouble et un support pharmaceutiquement acceptable.

7. Utilisation d'un composé ou sel suivant la revendication 1 dans la production d'un médicament pour le traitement : (a) d'une affection ou d'un trouble qui peut être traité en modulant la neurotransmission du glutamate chez un mammifère ; ou (b) d'une affection ou d'un trouble choisi entre un ictus, une ischémie cérébrale, un traumatisme de la moelle épinière, un traumatisme crânien, la maladie d'Alzheimer, la chorée de Huntington, la sclérose latérale amyotrophique, la démence induite par le SIDA, les spasmes musculaires, les céphalées de la migraine, l'incontinence urinaire, la psychose, les convulsions, l'hypoxie périnatale, une hypoxie, un arrêt cardiaque, l'altération neuronale hypoglycémique, des dépendances et accoutumances chimiques, l'altération oculaire et la rétinopathie, la maladie de Parkinson idiopathique et la maladie de Parkinson induite par un médicament, l'anxiété et un trouble panique, la schizophrénie, la dépression, un trouble bipolaire, le syndrome obsessionnel compulsif, le syndrome de Tourette, les vomissements, un oedème cérébral, la douleur chronique, et la douleur aiguë, la dyskinésie tardive et les déficiences cérébrales faisant suite à une intervention chirurgicale de dérivation cardiaque et une greffe cardiaque.

8. Composition pharmaceutique pour le traitement : (a) d'une affection ou d'un trouble qui peut être traité en modulant la neurotransmission du glutamate chez un mammifère ; ou (b) d'une affection ou d'un trouble choisi entre un ictus, une ischémie cérébrale, un traumatisme de la moelle épinière, un traumatisme crânien, la maladie d'Alzheimer, la chorée de Huntington, la sclérose latérale amyotrophique, la démence induite par le SIDA, les spasmes musculaires, les céphalées de la migraine, l'incontinence urinaire, la psychose, les convulsions, l'hypoxie périnatale, une hypoxie, un arrêt cardiaque, l'altération neuronale hypoglycémique des dépendances et accoutumances chimiques, l'altération oculaire et la rétinopathie, la maladie de Parkinson idiopathique et la maladie de Parkinson induite par un médicament, l'anxiété et un trouble panique, la schizophrénie, la dépression, un trouble bipolaire, le syndrome obsessionnel compulsif, le syndrome de Tourette, les vomissements, un oedème cérébral, la douleur chronique, et la douleur aiguë, la dyskinésie tardive et les déficiences cérébrales faisant suite à une intervention chirurgicale de dérivation cardiaque et une greffe cardiaque chez un mammifère, comprenant :
(a) un composé suivant la revendication 1 ou un de ses sels pharmaceutiquement acceptables ;
(b) un inhibiteur de réabsorption de sérotonine ou un ligand du récepteur 5HT_{1A}, ou un sel pharmaceutiquement acceptable de cet inhibiteur ou ligand ; et
(c) un support pharmaceutiquement acceptable ;
dans lequel le composé suivant la revendication 1 et le ligand du récepteur de 5HT_{1A} ou l'inhibiteur de réabsorption de sérotonine qui sont présents dans cette composition sont présents en des quantités telles que l'association des deux ingrédients actifs soit efficace dans le traitement de cette affection ou de ce trouble.

9. Utilisation (i) d'un composé ou sel suivant la revendication 1 et (ii) d'un inhibiteur de réabsorption de sérotonine ou d'un ligand de 5HT_{1A} ou d'un sel pharmaceutiquement acceptable de cet inhibiteur ou ligand, dans la production d'un médicament pour le traitement :
(a) d'une affection ou d'un trouble qui peut être traité en modulant la neurotransmission du glutamate chez un mammifère ; ou (b) d'une affection ou d'un trouble choisi entre un ictus, une ischémie cérébrale, un traumatisme de la moelle épinière, un traumatisme crânien, la maladie d'Alzheimer, la chorée de Huntington, la sclérose latérale amyotrophique, la démence induite par le SIDA, les spasmes musculaires, les céphalées de la migraine, l'incontinence urinaire, la psychose, les convulsions, l'hypoxie périnatale, une hypoxie, un arrêt cardiaque, l'altération neuronale hypoglycémique des dépendances et accoutumances chimiques, l'altération oculaire et la rétinopathie, la maladie de Parkinson idiopathique et la maladie de Parkinson induite par un médicament, l'anxiété et un trouble panique, la schizophrénie, la dépression, un trouble bipolaire, le syndrome obsessionnel compulsif, le syndrome de Tourette, les vomissements, un oedème cérébral, la douleur chronique, et la douleur aiguë, la dyskinésie tardive et les déficiences cérébrales faisant suite à une intervention chirurgicale de dérivation cardiaque et une greffe cardiaque.

10. Composition pharmaceutique comprenant un composé ou sel suivant l'une quelconque des revendications 1 à 6 et un support pharmaceutiquement acceptable.

11. Composé ou sel suivant l'une quelconque des revendications 1 à 6, destiné à être utilisé en médecine.
